# EUROPEAN PATENT SPECIFICATION

(11) **EP 1 866 303 B1**
(45) Date of publication and mention of the grant of the patent: **27.11.2013**
(21) Application number: 05714624.3
(22) Date of filing: 14.03.2005
(51) Int. Cl.: C07D 411/04

(54) **PROCESS AND METHODS FOR THE PREPARATION OF OPTICALLY ACTIVE CIS-2-HYDROXYMETHYL-4-(CYTOSIN-1-YL)-1,3-OXATHIOLANE OR PHARMACEUTICALLY ACCEPTABLE SALTS THEREOF**
VERFAHREN UND METHODEN ZUR HERSTELLUNG VON OPTISCH AKTIVEM CIS-2-HYDROXYMETHYL-4-(CYTOSIN-1-YL)-1,3-OXATHIOLAN ODER PHARMAZEUTISCH UNBEDENKLICHEN SALZEN DAVON
PROCEDES DE PREPARATION DU CIS-2-HYDROXYMETHYL-4-(CYTOSIN-1 -YL)-1,3-OXATHIOLANE OPTIQUEMENT ACTIF OU DE SELS DE CELUI-CI ACCEPTABLES SUR LE PLAN PHARMACEUTIQUE

(43) Date of publication of application: 19.12.2007
(73) Proprietor: Shire Canada Inc., Ville St-Laurent, QC H4S 2C9 (CA)
(72) Inventor: CIMPOIA, Alex, Verdun, Québec H3E 1C9 (CA); SIMION, Dan., Deceased (CA)
(74) Representative: Dey, Michael
(86) International application number: PCT/CA2005/000384
(87) International publication number: WO 2006/096954

(56) References cited:
- WO-A1-95/29176
- CA-A1- 2 095 613
- CA-A1- 2 188 283
- US-A1- 2003 013 880
- Bayley C.R. and Vaidya N.A.: "Resolution of Racemates by Diastereomeric Salt formation"; "Chapter 2" In: Collins A.N., Sheldrake G. N., Crosby J.: "Chirality in Industry" 1992, John Wiley and Sons Ldt. , Chichester, England , XP002577078 , pages 69-77 * Whole document, specially page 69, second paragraph and pages 72-73 *
- WEI WANG ET AL: "SYNTHESIS OF OPTICALLY ACTIVE 2',3'-DIDEOXY-3'-OXA-4'-THIO-RIBONUCLE OSIDE ANALOGUES BY TRANSPOSITION OF A LEAVING GROUP ON CHIRAL OXATHIOLANES VIA A REDUCTIVE-OXIDATIVE PROCESS" TETRAHEDRON LETTERS, ELSEVIER, AMSTERDAM, NL LNKD- DOI:10.1016/S0040-4039(00)76955-3, vol. 35, no. 27, 4 July 1994 (1994-07-04), pages 4739-4742, XP002178186 ISSN: 0040-4039
- WANG W. ET AL. TETRAHEDRON LETTERS vol. 35, no. 27, 04 July 1994, pages 4739 - 4742, XP002178186
- BELLEAU B. ET AL. BIOORG. MED. CHEM. LET. vol. 3, no. 8, August 1993, pages 1723 - 1728, XP001024523

## Description

### FIELD OF INVENTION

The present invention relates to the field of making optically active compounds, particularly the preparation of optically active.oxathiolane nucleosides.

### BACKGROUND

Classes of compounds known as 2-substituted-4-substituted-1,3-oxathiolanes have been found to have potent antiviral activity. In particular, these compounds have been found to act as potent inhibitors of HIV-1 replication in T-lymphocytes over a prolonged period of time with less cytotoxic side effects than compounds known in the art. These compounds have also been found active against 3TC-resistant HIV strains. These compounds are also useful in prophylaxis and treatment of hepatitis B virus infections.

Cis-2-Hydroxymethyl-4-(cytosin-1'-yl)-1,3-oxathiolane can be produced by the methods described by Mansour et al., "AntiHuman Immunodeficiency Virus and Anti-Hepatitis-B Virus Activities and Toxicities of the Enantiomers of 2'-Deoxy-3'-oxa-4'-thiacytidine and Their 5-Fluoro Analogues in vitro", J. Med. Chem., (1995), Vol. 38, No. 1, pp. 1-4, as well as US 6,228,860 or Nucleosides and Nucleotides, (1995) 14(3-5) pp. 627-735.

Typically, when compounds are desired as a single enantiomer they may be obtained either by resolution of the mixture of the two cis enantiomers by chiral HPLC or by stereospecific synthesis from isometrically pure starting material or any convenient intermediate. A complete review of known technology may be found in "Enantiomers, Racemates and Resolutions" by J. Jacques, A. Collet & S.H. Wilen (John Wiley & Sons, 1981). Alternatively, compounds or any convenient intermediate may be resolved by enzymatic resolution with a suitable enzyme such as cytidine deaminase or selective enzymatic degradation of a suitable derivative. See for example Storer et al., "The resolution and Absolute Stereochemistry of the Enantiomers of cis-1[2(Hydroxymethyl)-1,3-Oxathiolan-5-yl)Cytosine (BCH-189): Equipotent Anti-HIV Agents", Nucleosides & Nucleotides, 12(2), 225-236 (1993).

Another process known as resolution by formation of diastereomeric compounds require intervention of chiral agents. Unlike enantiomers, diastereomers may have significantly different physico-chemical properties that may allow for the separation from one another. One variation of such technique involves the formation and separation of diastereomeric salts between a racemic substance and an optically active resolving acid or base. Pasteur first reported the resolution of a racemic acid using an optically active base (Pasteur, L., C.R Acad. Sci. (1853) 37 p.162; Pasteur, L., Ann. Chim (Paris) (1853) 3, 38 p. 437). A resolution using nonstochiometric quantities of chiral agents was studied by Marckwald 1896 and later referred to as "method of half-quantity" (Marckwald, W., Ber. (1896), 29, p. 42; Marckwald, W., Ber. (1896), 29, p. 43). The process for the resolution of tartaric acid through crystallization of its salt of cinchonine was improved by Marckwald while using only half of the cinchonine necessary for formation of the tartrate salt. The resolution is based on the separation of one of the diastereomers and one of the enantiomers rather than the separation of two diastereomeric salts formed in equal quantities. When using the method of half-quantity, the racemate is partially neutralized by the optically active resolving agent. In the process described by Pope & Peachey (Pope, W.J., Peachey,S.J. J, Chem. Soc. (1899) 75, p.1066) the excess of racemate not neutralized by the resolving agent is neutralized by the addition of the necessary quantity of an achiral acid or base (depending on whether the resolving agent was an acid or base).

US 2003/013880 A1 relates to a process for recovery of cis-1,3-oxathiolane nucleosides from their undesired trans-isomers.

Bayley, C.R. and Vaidya, N.A. (in: "Resolution of Racemates by Diastereomeric Salt formation"; Chapter 2 in: Collins, A.N., Sheldrake, G.N., Crosby, J.: "Chirality in Industry" 1992, John Wiley & Sons Ltd., pp. 69-77) discuss the industrial application of diastereomeric crystallization and the variables affecting the separation of enantiomers.

Wang et al., Tetrahedron Letters, Vol. 35, No. 27, pp. 4739-4742, 1994 describes the synthesis of chiral nucleoside analogs and mentions resolution of enantiomers by chiral HPLC.

WO 95/29176 relates to single enantiomers of cis-substituted 1,3-oxathiolanes. Separation of single enantiomers may be achieved either by resolution of the mixture of two cis-enantiomers (by chiral HPLC) or by stereospecific synthesis (cf. page 13, lines 5-8).

Belleau, B. et al., Bioorg. Meth. Chem. Let., vol. 3, no. 8, 1993, pp. 1723-1728 describe a novel class of 1,3-oxathiolane nucleoside analogs having potent anti-HIV activity.

### SUMMARY OF THE INVENTION

In one aspect, the present invention relates to a process for producing crystalline optically active cis-2-hydroxymethyl-4-(cytosin-1'-yl)-1,3-oxathiolane, comprising:
a) reacting cis-2-hydroxymethyl-4-(cytosin-1'-yl)-1,3-oxathiolane with a chiral acid and an achiral acid to produce one diastereomeric salt and one enantiomeric salt;
b) recovering said diastereomeric salt;
c) converting said diastereomeric salt into optically active cis-2-hydroxymethyl-4-(cytosin-1'-yl)-1,3-oxathiolane,
wherein said chiral acid is (1 R)-(-)-10-camphorsulfonic acid, and
wherein said diastereomeric salt is (-)-cis-2-hydroxymethyl-4-(cytosin-1'-yl)-1,3-oxathiolane•(1R)-(-)-10-camphorsulfonate. In a further aspect, the present invention further provides novel cis-2-hydroxymethyl-4-(cytosin-1'-yl)-1,3-oxathiolane salts.

Further disclosed is a process for producing optically active compound of formula I or II: 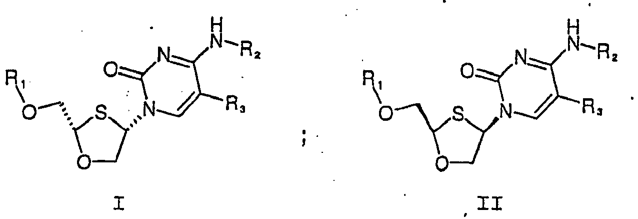 wherein:
- R₁: is H, C₁₋₆ alkyl, C₆₋₁₂ aryl, C₆₋₁₂ arylalkyl (e.g., C₇₋₁₂ arylalkyl), (CO)C₁₋₆ alkyl, (CO)O-C₁₋₆ alkyl, (CO)C₆₋₁₂ aryl, or (CO)C₆₋₁₂ arylalkyl (e.g., (CO) C₇₋₁₂ arylalkyl);
- R₂: is H, C₁₋₆ alkyl or CO-R₅; wherein R₅ is H or C₁₋₆ alkyl;
- R₃: is H, C₁₋₆ alkyl, bromide, chloride, fluoride, iodide or CF₃; comprising:

a) reacting a compound of formula III in the cis configuration: 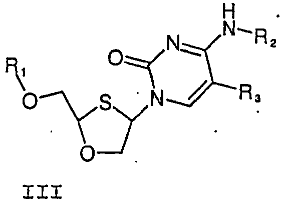 with a chiral acid to produce two diastereomeric salts;
b) recovering substantially one diastereomeric salt;
c) converting said one diastereomeric salt into said optically active compound.

Also disclosed is a method for resolving a compound of formula III, in the cis configuration: 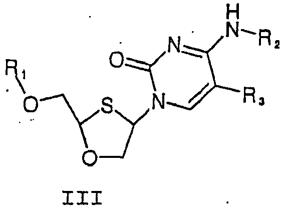 wherein:
- R₁: is H, C₁₋₆ alkyl, C₆₋₁₂ aryl, C₆₋₁₂ arylalkyl (e.g., C₇₋₁₂ arylalkyl), (CO)C₁₋₆ alkyl, (CO)O-C₁₋₆ alkyl, (CO)O-C₆₋₁₂ aryl, or (CO)-C₆₋₁₂ arylalkyl (e.g., (CO)C₇₋₁₂ arylalkyl);
- R₂: is chosen from H, C₁₋₆ alkyl, C₁₋₆ acyl and CO-R₅;
wherein R₅ is H or C₁₋₆ alkyl;
- R₃: is H, C₁₋₆ alkyl, bromide, chloride, fluoride, iodide or CF₃; comprising:

a) reacting said compound of formula III with a chiral acid to produce two diastereomeric salts;
b) recovering substantially one diastereomeric salt;
c) converting said one diastereomeric salt into compound of formula I or II:

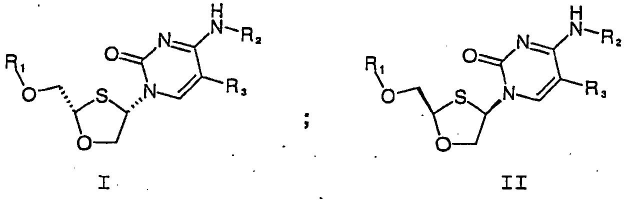

Also disclosed is a process for producing optically active cis-2-hydroxymethyl-4-(cytosin-1'-yl)-1,3-oxathiolane, comprising:
a) reacting a chiral acid with cis-2-hydroxymethyl-4-(cytosin-1'-yl)-1,3-oxathiolane to produce two diastereomeric salts;
b) recovering substantially one diastereomeric salt;
c) converting said one diastereomeric salt into said optically active cis-2-hydroxymethyl-4-(cytosin-1'-yl)-1,3-oxathiolane.

Also disclosed is a process for producing (-)-cis-2-hydroxymethyl-4-(cytosin-1'-yl)-1,3-oxathiolane comprising:
a) reacting cis-2-hydroxymethyl-4-(cytosin-1'-yl)-1,3-oxathiolane with a half quantity molar amount of a chiral acid to substantially produce (-)-cis-2-hydroxymethyl-4-(cytosin-1'-yl)-1,3-oxathiolane•chiral acid salt, said molar ratio being with regard to cis-2-hydroxymethyl-4-(cytosin-1'-yl)-1,3-oxathiolane;
b) recovering said (-)-cis-2-hydroxymethyl-4-(cytosin-1'-yl)-1,3-oxathiolane•chiral acid salt;
c) converting said (-)-cis-2-hydroxymethyl-4-(cytosin-1'-yl)-1,3-oxathiolane•chiral acid salt into said (-)-cis-2-hydroxymethyl-4-(cytosin-1'-yl)-1,3-oxathiolane.

Also disclosed is a process for producing optically active cis-2-hydroxymethyl-4-(cytosin-1'-yl)-1,3-oxathiolane, comprising:
a) reacting cis-2-hydroxymethyl-4-(cytosin-1'-yl)-1,3-oxathiolane with a chiral acid and an achiral acid to produce substantially one diastereomeric salt and substantially one enantiomeric salt;
b) recovering said diastereomeric salt;
c) converting said diastereomeric salt into optically active cis-2-hydroxymethyl-4-(cytosin-1'-yl)-1,3-oxathiolane.

### DETAILED DESCRIPTION OF THE INVENTION

Accordingly, the present invention relates to a process for producing crystalline optically active cis-2-hydroxymethyl-4-(cytosin-1'-yl)-1,3-oxathiolane, comprising:
a) reacting cis-2-hydroxymethyl-4-(cytosin-1'-yl)-1,3-oxathiolane with a chiral acid and an achiral acid to produce one diastereomeric salt and one enantiomeric salt;
b) recovering said diastereomeric salt;
c) converting said diastereomeric salt into optically active cis-2-hydroxymethyl-4-(cytosin-1'-yl)-1,3-oxathiolane,
wherein said chiral acid is (1R)-(-)-10-camphorsulfonic acid, and
wherein said diastereomeric salt is (-)-cis-2-hydroxymethyl-4-(cytosin-1'-yl)-1,3-oxathiolane•(1R)-(-)-10-camphorsulfonate. In a further aspect, the present invention further provides novel cis-2-hydroxymethyl-4-(cytosin-1'-yl)-1,3-oxathiolane salts.

Further disclosed is a process for producing optically active compound of formula I or II: 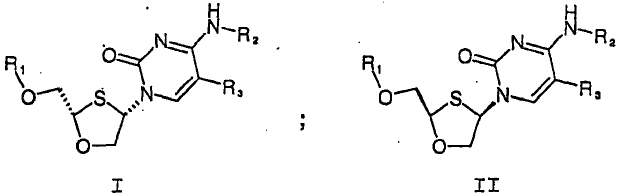 wherein:
R₁ is H, C₁₋₆ alkyl, C₆₋₁₂ aryl, C₆₋₁₂ arylalkyl (e.g., C₇₋₁₂ arylalkyl), (CO)C₁₋₆ alkyl, (CO)O-C₁₋₆ alkyl, (CO)C₆₋₁₂ aryl, or (CO)C₆₋₁₂ arylalkyl (e.g., (CO)C₇₋₁₂ arylalkyl);
R₂ is H, C₁₋₆ alkyl or CO-R₅;
wherein R₅ is H or C₁₋₆ alkyl;
R₃ is H, C₁₋₆ alkyl, bromide, chloride, fluoride, iodide or CF₃; comprising:
   a) reacting a compound of formula III in the cis configuration: 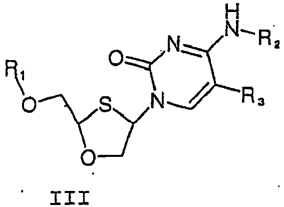 with a chiral acid to produce two diastereomeric salts;
   b) recovering substantially one diastereomeric salt;
   c) converting said one diastereomeric salt into said optically active compound.

The scope the present invention includes a process as described above wherein the over-all yield of the desired enantiomer is equal to or greater than 25% (100g of racemate would produce at least 25g of the desired enantiomer).

An embodiment of the present invention relates to a process which generates a final product which is substantially in the form of a single enantiomer. Additionally, an embodiment of the present invention includes a process described above which results in a product having an enantiomeric excess of 99% or higher.

Also disclosed is a process for producing optically active compound of formula I or II: 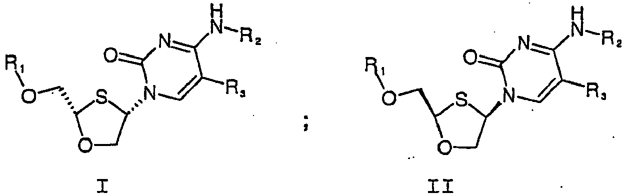 wherein: R₁, R₂, R₃ are as defined above, comprising:
a) reacting a compound of formula III in the cis configuration: 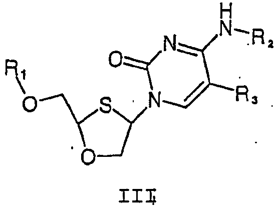 with a chiral acid to produce two diastereomeric salts;
b) recovering substantially one diastereomeric salt;
c) converting said one diastereomeric salt into said optically active compound.

There is also disclosed a method for resolving a compound of formula III, in the cis configuration: 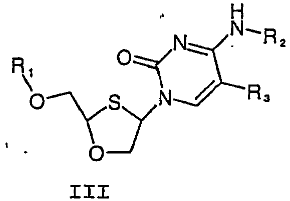 wherein: R₁, R₂, R₃ are as defined above, comprising:
a) reacting said compound of formula III with a chiral acid to produce two diastereomeric salts;
b) recovering substantially one diastereomeric salt;
c) converting said one diastereomeric salt into a compound of formula I or II:

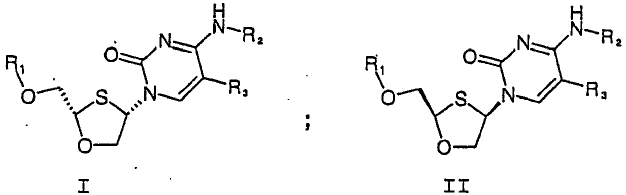

It is also disclosed that R₁ is H, C₁₋₆ alkyl, (CO) C₁₋₆ alkyl, (CO) O-C₁₋₆ alkyl or (CO)C₆₋₁₂ aryl.

It is also disclosed that
R₁ is H, (CO)C₁₋₆ alkyl or (CO)C₆₋₁₂ aryl,
R₁ is H,
R₁ is (CO) C₆₋₁₂ aryl.

Still, further, it is disclosed that
R₂ is H or C₁₋₆ alkyl,
R₂ is CO-R₅, wherein R₅ is H or C₁₋₆ alkyl,
R₂ is formyl or acetyl.

It is also disclosed that R₃ is H, C₁₋₆ alkyl or fluoride.
In further embodiments:
R₃ is H or fluoride,
R₃ is H,
R₃ is fluoride.

It is also disclosed that the chiral acid is (1R)-(-)-10-camphorsulfonic acid, (-)-2,3-dibenzoyl-L-tartaric acid, (+)-L-tartaric acid or (-)-L-malic.acid.

It is also disclosed that the chiral acid is (1R)-(-)-10-camphorsulfonic acid.

It is also disclosed that the optically active compound is

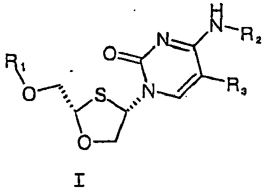

It is also disclosed that the optically active compound is

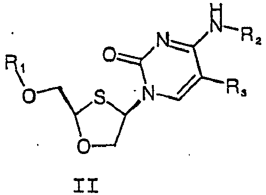

It is also disclosed that the two diastereomeric salts comprise a first more soluble diastereomeric salt and a second less soluble diastereomeric salt.

It is also disclosed that step b) described above further comprises recovering a second diastereomeric salt.

Also disclosed is a process for producing optically active compound of formula IV: 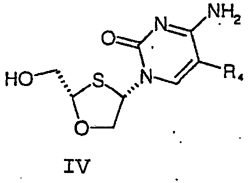 wherein:
R₄ is H or fluoride; comprising:
   a) reacting a compound of formula III in the cis configuration: 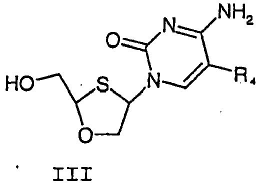 with a chiral acid selected from (lR)-(-)-10-camphorsulfonic acid, (-)-2,3-dibenzoyl-L-tartaric acid, (+)-L-tartaric acid or (-)-L-malic acid, to produce two diastereomeric salts;
   b) recovering substantially one diastereomeric salt;
   c) converting said one diastereomeric salt into said optically active compound.

Also disclosed is a process for producing optically active cis-2-hydroxymethyl-4-(cytosin-1'-yl)-1,3-oxathiolane, comprising:
a) reacting a chiral acid with cis-2-hydroxymethyl-4-(cytosin-1'-yl)-1,3-oxathiolane to produce two diastereomeric salts;
b) recovering substantially one diastereomeric salt;
c) converting said one diastereomeric salt into said optically active cis-2-hydroxymethyl-4-(cytosin-1'-yl)-1,3-oxathiolane.

It is also disclosed that the optically active cis-2-hydroxymethyl-4-(cytosin-1'-yl)-1,3-oxathiolane is crystalline.

It is also disclosed that the optically active cis-2-hydroxymethyl-4-(cytosin-1'-yl)-1,3-oxathiolane is the (-) enantiomer.

It is also disclosed that the optically active cis-2-hydroxymethyl-4-(cytosin-1'-yl)-1,3-oxathiolane is the (+) enantiomer.

It is also disclosed that the chiral acid is (1R)-(-)-10-camphorsulfonic acid, (-)-2,3-dibenzoyl-L-tartaric acid, (+)-L-tartaric acid or (-)-L-malic acid.

It is also disclosed that the chiral acid is (1R)-(-)-10-camphorsulfonic acid.

It is also disclosed that the optically active cis-2-hydroxymethyl-4-(cytosin-1'-yl)-1,3-oxathiolane has an enantiomeric excess of 60% or higher.

It is also disclosed that the optically active cis-2-hydroxymethyl-4-(cytosin-1'-yl)-1,3-oxathiolane has an enantiomeric excess of 70% or higher.

It is also disclosed that the optically active cis-2-hydroxymethyl-4-(cytosin-1'-yl)-1,3-oxathiolane has an enantiomeric excess of 80% or higher.

It is also disclosed that the optically active cis-2-hydroxymethyl-4-(cytosin-1'-yl)-1,3-oxathiolane has an enantiomeric excess of 90% or higher.

In one embodiment, the optically active cis-2-hydroxymethyl-4-(cytosin-1'-yl)-1,3-oxathiolane has an enantiomeric excess of 95% or higher.

In one embodiment, the optically active cis-2-hydroxymethyl-4-(cytosin-1'-yl)-1,3-oxathiolane has an enantiomeric excess of 98% or higher.

In one embodiment, the optically active cis-2-hydroxymethyl-4-(cytosin-1'-yl)-1,3-oxathiolane has an enantiomeric excess.of 99% or higher.

Also disclosed is a process for producing (-)-cis-2-hydroxymethyl-4-(cytosin-1'-yl)-1,3-oxathiolane•chiral acid salt, comprising:
a) reacting cis-2-hydroxymethyl-4-(cytosin-1'-yl)-1,3-oxathiolane with a chiral acid to.produce (-)-cis-2-hydroxymethyl-4-(cytosin-1'-yl)-1,3-oxathiolane•chiral acid salt and (+)-cis-2-hydroxymethyl-4-(cytosin-1'-yl)-1,3-oxathiolane•chiral acid salt;
b) recovering said (-)-cis-2-hydroxymethyl-4-(cytosin-1'-yl)-1,3-oxathiolane•chiral acid salt.

It is also disclosed that said step b) further comprises recovering (+)-cis-2-hydroxymethyl-4-(cytosin-1'-yl)-1,3-oxathiolane•chiral acid salt.

It is also disclosed that said (-)-cis-2-hydroxymethyl-4-(cytosin-1'-yl)-1,3-oxathiolane.chiral acid salt contains less than 30% of (+)-cis-2-hydroxymethyl-4-(cytosin-1'-yl)-1,3-oxathiolane•chiral acid salt.

**It is also disclosed that:**
(-)-cis-2-hydroxymethyl-4-(cytosin-1'-yl)-1,3-oxathiolane•chiral acid salt contains less than 20% of (+)-cis-2-hydroxymethyl-4-(cytosin-1'-yl)-1,3-oxathiolane•chiral acid salt;
(-)-cis-2-hydroxymethyl-4-(cytosin-1'-yl)-1,3-oxathiolane•chiral acid salt contains less than 10% of (+)-cis-2-hydroxymethyl-4-(cytosin-1'-yl)-1,3-oxathiolane•chiral acid salt;
(-)-cis-2-hydroxymethyl-4-(cytosin-1'-yl)-1,3-oxathiolane•chiral acid salt contains less than 5% of (+)-cis-2-hydroxymethyl-4-(cytosin-1'-yl)-1,3-oxathiolane•chiral acid salt;
(-)-cis-2-hydroxymethyl-4-(cytosin-1'-yl)-1,3-oxathiolane•chiral acid salt contains less than 1% of (+)-cis-2-hydroxymethyl-4-(cytosin-1'-yl)-1,3-oxathiolane•chiral acid salt;
(-)-cis-2-hydroxymethyl-4-(cytosin-1'-yl)-1,3-oxathiolane•chiral acid salt is substantially free of (+)-cis-2-hydroxymethyl-4-(cytosin-1'-yl)-1,3-oxathiolane•chiral acid salt.

It is also disclosed that the process further comprises recrystallizing said (-)-cis-2-hydroxymethyl-4-(cytosin-1'-yl)-1,3-oxathiolane•chiral resolving acid addition salt.

It is also disclosed that said chiral acid is in stoichiometric molar ratio with regard cis-2-hydroxymethyl-4-(cytosin-1'-yl)-1,3-oxathiolane.

It is also disclosed that said chiral acid is in nonstoichiometric molar ratio with regard to cis-2-hydroxymethyl-4-(cytosin-1'-yl)-1,3-oxathiolane.

In one embodiment of the process according to claim 1 of the appended claim set, cis-2-hydroxymethyl-4-(cytosin-1'-yl)-1,3-oxathiolane is reacted with a half-quantity molar amount of a chiral acid, said molar ratio being with regard to cis-2-hydroxymethyl-4-(cytosin-1'-yl)-1,3-oxathiolane.

In one embodiment, said step a) further comprise adding a half-quantity molar amount of achiral acid to substantially produce (+)-cis-2-hydroxymethyl-4-(cytosin-1'-yl)-1,3-oxathiolane•achiral acid salt, said molar ratio being with regard to cis-2-hydroxymethyl-4-(cytosin-1'-yl)-1,3-oxathiolane.

It is also disclosed that said chiral acid is (1R)-(-)-10-camphorsulfonic acid, (-)-2,3-dibenzoyl-L-tartaric acid, (+)-L-tartaric acid or (-)-L-malic acid.

In one embodiment, said chiral acid is (1R)-(-)-10-camphorsulfonic acid.

In one embodiment, said achiral acid is hydrochloric acid.

It is also disclosed that there is provided a process for producing crystalline optically active cis-2-hydroxymethyl-4-(cytosin-1'-yl)-1,3-oxathiolane, comprising:
a) reacting cis-2-hydroxymethyl-4-(cytosin-1'-yl)-1,3-oxathiolane with a chiral acid and an achiral acid to produce substantially one diastereomeric salt and substantially one enantiomeric salt;
b) recovering said diastereomeric salt;
c) converting said diastereomeric salt into optically active cis-2-hydroxymethyl-4-(cytosin-1'-yl)-1,3-oxathiolane.

An embodiment of the process of the present invention generates an over-all yield equal to or greater than 25% of the desired enantiomer.

An additional embodiment of the process of the present invention generates the desired enantiomer with an enantiomeric excess of 95% or higher.

Another embodiment of the process of the present invention generates an over-all yield equal to or greater than 25% of the desired enantiomer and an enantiomeric excess of 99% or higher.

In one embodiment, said diastereomeric salt is (-)-cis-2-hydroxymethyl-4-(cytosin-1'-yl)-1,3-oxathiolane.(1R)-(-)-10-camphorsulfonate.

It is also disclosed that said enantiomeric salt is (+)-cis-2-hydroxymethyl-4-(cytosin-1'-y1)-1,3-oxathiolane.hydrochloric acid salt.

An "oxathiolane ring" is any substituted or unsubstituted five member monocyclic ring that has an oxygen atom in position 1 and a sulfur atom in position 3 of the ring as illustrated below:

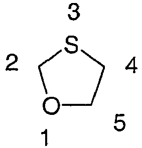

It will be apparent to a skilled person in the field that the reaction conditions described in these examples may be modified and still achieve successful results.

Typically, solvents, temperature and time of reaction may be varied. A suitable solvent will allow the process to occur under the reaction conditions without adversely affecting the reaction. The solvent may be one or more solvents and may be organic (e.g., methanol, ethanol, propanol, isopropanol, dichloromethane, dichloroethane, tetrahydrofuran, hexane, pentane, ether spirit, ethyl ether), water or aqueous/organic (e.g., methanol-water, isopropanol-water). The solvents may also be present in various ratios (for example 1:1, 2:1, 5:1, 10:1 or 1:1:1, 1:2:1).

The temperature may be varied and will allow the process to occur under the reaction conditions. The suitable temperature will provide the desired product without adversely affecting the reaction.

It will be appreciated by a person of skill in the art that a suitable period of time is a time for obtaining a sufficient chemical transformation of the starting material, obtaining the desired purity or the desired yield of the reaction product or a combination of those. The reaction can typically be monitored, if desired, by thin layer chromatography, light absorption (e.g., U.V.) of reaction medium, gas chromatography or high performance liquid chromatography (HPLC).

Cis-2-Hydroxymethyl-4-(cytosin-1'-yl)-1,3-oxathiolane exist as enantiomers which may be in various ratios.

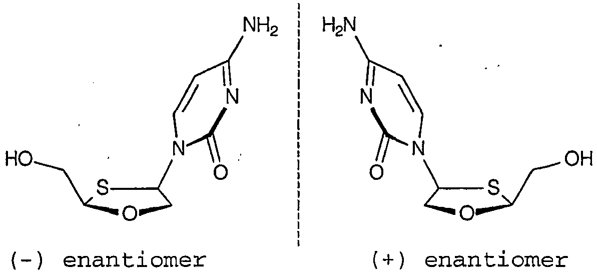

For example the enantiomers may be present as racemate (i.e. in equal proportions) or any alternative ratio of the enantiomers such as for example 1:1, 2:1, 5:1, 10:1, 100:1 or 1:2, 1:5, 1:10, 1:100. References hereinafter to cis-2-Hydroxymethyl-4-(cytosin-1'-yl)-1,3-oxathiolane according to the invention includes all such possible ratios of enantiomers.

Unless otherwise defined, all technical and scientific terms used herein have the same meaning as commonly understood by one of ordinary skill in the art to which this invention belongs. In case of conflict, the present specification, including definitions, will control. In addition, the materials, methods, and examples are illustrative only and not intended to be limiting.

As used in this application, the term "alkyl" represents a straight chain or branched chain hydrocarbon moiety which may optionally be substituted by one or more of halogen, nitro, nitroso, sulfate, sulfate ester, sulfonate, sulfonate ester, phosphonate ester, amide, C₁₋₆ alkyl, C₆₋₁₂ aralkyl (e.g., C₇₋₁₂ aralkyl), C₆₋₁₂ aryl, C₁₋₆ alkyloxy, C₆₋₁₂ aryloxy, C(O)-C₁₋₆ alkyl, C(O)-C₆₋₁₂ aryl, C(O)C₆₋₁₂ aralkyl (e.g., C(O)C₇₋₁₂ aralkyl), heterocycle having 3-10 ring-members, hydroxyl, amino, ester, cyano, azido, amidino or guanido. Useful examples of alkyl include isopropyl, propyl, ethyl, methyl, hexyl or cyclopropyl, which in each case is unsubstituted or substituted one or more times by, for example, halogen, nitro, nitroso, sulfate, sulfonate, amide, hydroxyl, amino, ester, cyano, azido, amidino or guanido. The term alkyl is also meant to include alkyl in which one or more hydrogen atoms are each replaced by a halogen, preferably fluoro (e.g., CF₃- or CF₃CH₂-).

The term "aryl" represents a carbocyclic moiety containing at least one benzenoid-type ring which may optionally be substituted by one or more of halogen, nitro, nitroso, sulfate, sulfate ester, sulfonate, sulfonate ester, phosphonate ester, amide, C₁₋₆ alkyl, C₆₋₁₂ aralkyl, C₆₋₁₂ aryl, C₁₋₆ alkyloxy, C₆₋₁₂ aryloxy, C(O)-C₁₋₆ alkyl, C(O)-C₆₋₁₂ aryl, C(O)C₆₋₁₂ aralkyl, heterocycle having 3-10 ring-members, hydroxyl, amino, ester, cyano, azido, amidino or guanido. Examples of aryl include phenyl and naphthyl, which in each case is unsubstituted or substituted one or more times by, for example, halogen, nitro, nitroso, sulfate, sulfonate, amide, hydroxyl, amino, ester, cyano, azido, amidino or guanido.

The term "aralkyl" represents an aryl group attached to the adjacent atom by an alkyl. Useful examples include benzyl which is unsubstituted or substituted one or more times by, for example, halogen, nitro, nitroso, sulfate, sulfonate, amide, hydroxyl, amino, ester, cyano, azido, amidino or guanido.

The term "heterocycle" represents a saturated or unsaturated, cyclic moiety wherein said cyclic moiety is interrupted by at least one heteroatom, (e.g., oxygen, sulfur or nitrogen) which may optionally be substituted by one or more of halogen, nitro, nitroso, sulfate, sulfate ester, sulfonate, sulfonate ester, phosphonate ester, amide, C₁₋₆ alkyl, C₆₋₁₂ aralkyl, C₆₋₁₂ aryl, C₁₋₆ alkyloxy, C₆₋₁₂ aryloxy, C(O)-C₁₋₆ alkyl, C(O)-C₆₋₁₂ aryl, C(O)C₆₋₁₂ aralkyl, hydroxyl, amino, ester, cyano, azido, amidino or guanido. It is understood that the term heterocyclic ring represents a mono or polycyclic (e.g., bicyclic) ring. Examples of heterocyclic rings include but are not limited to epoxide; furan; benzofuran; isobenzofuran; oxathiolane; dithiolane; dioxolane; pyrrole; pyrrolidine; imidazole; pyridine; pyrimidine; indole; piperidine; morpholine; thiophene and thiomorpholine, which in each case is unsubstituted or substituted one or more times by, for example, halogen, nitro, nitroso, sulfate, sulfonate, amide, hydroxyl, amino, ester, cyano, azido, amidino or guanido.

The term "independently" means that a substituent can be the same or different definition for each item.

The term "optically active" means that the enantiomeric excess is greater than zero.

The optical purity is numerically equivalent to the "enantiomeric excess". The term "enantiomeric excess" or "ee" is defined in percentage (%) value as follows: [mole fraction (major enantiomer) - mole fraction (minor enantiomer)] x 100. (for example, an ee of 99% represent a ratio of 99.5% of one enantiomer and 0.5% of the opposite enantiomer).

The term "chiral acid" means an optically active acidic compound able to form a diastereomer with a compound of formula III, such as 2-hydroxymethyl-4-(cytosin-1'-yl)-1,3-oxathiolane. Examples of such acids include without limitation: tartaric acid, O,O'-dibenzoyltartaric acid, O,O'-di-p-toluoyltartaric acid, 2-nitrotartranilic acid, mandelic acid, malic acid, 2-phenoxypropionic acid, 10-camphorsulfonic acid, hydratropic acid, N-acetylleucine, N-(α-methylbenzyl)succinamic acid, N-(α-methylbenzyl)phthamic acid, 3-bromocamphor-9-sulfonic acid, camphor-3-sulfonic acid, quinic acid, di-O-isopropylidene-2-oxo-L-gulonic acid, lasalocid, 1,1'-binaphthyl-2,2/-phosphoric acid, cholestenonesulfonic acid. Further examples include (1R)-(-)-10-camphorsulfonic acid, (-)-2,3-dibenzoyl-L-tartaric acid, (+)-L-tartaric acid and (-)-L-malic acid.

A person of ordinary skill will understand that the term "achiral acid" includes a variety of acids such as inorganic acids (e.g., HCl, HBr, H₂SO₄, HBF₄); sulfonic acids (e.g., methanesulfonic, benzenesulfonic, p-toluenesulfonic, p-hydroxytoluenesulfonic, sulfanilic, p-chlorobenezenesulfonic); substituted acetic acids (e.g., glycolic, chloro-, dichloro-, trichloroacetic); polycarboxylic and oxy acids (e.g., succinic, adipic, maleic, fumaric, citric, pyruvic):

Described are also pharmaceutically acceptable salts of the compounds described herein. By the term "pharmaceutically acceptable salts" is meant salts derived from pharmaceutically acceptable inorganic and organic acids and bases. Examples of suitable acids include hydrochloric, hydrobromic, sulphuric, nitric, perchloric, fumaric, maleic, phosphoric, glycollic, lactic, salicylic, succinic, toleune-p-sulphonic, tartaric, acetic, trifluoroacetic, citric, methanesulphonic, formic, benzoic, malonic, naphthalene-2-sulphonic, cysteic acid and benzenesulphonic acids. Other acids such as oxalic, while not themselves pharmaceutically acceptable, may be useful as intermediates in obtaining the compounds of the invention and their pharmaceutically acceptable acid addition salts.
Salts derived from appropriate bases include alkali metal (e.g., sodium), alkaline earth metal (e.g., magnesium), ammonium and NR₄⁺ (where R is C₁₋₄ alkyl) salts.

Dowex® Marathon A-OH is a mark of DOW Chemical Company.

In one aspect, the present invention provides novel compounds as described in table 1:

**TABLE 1**

| | |
|---|---|
| 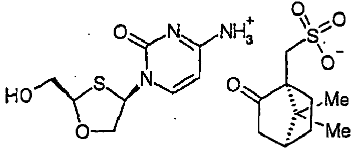 | (-)-cis-2-hydroxymethyl-4-(cytosin-1'-yl)-1,3-oxathiolane.(1R)-(-) -10-camphorsulfonate |
| 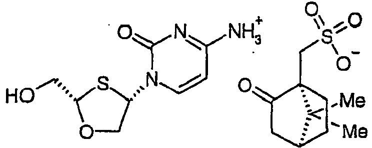 | (+)-cis-2-hydroxymethyl-4-(cytosin-1'-yl)-1,3-oxathiolane.(1R)-(-)-10-camphorsulfonate |
| 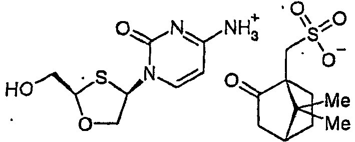 | (-)-cis-2-hydroxymethyl-4-(cytosin-1'-yl)-1,3-oxathiolane.(1S)-(+)-10-camphorsulfonate |
| 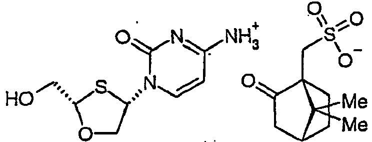 | (+)-cis-2-hydroxymethyl-4-(cytosin-1'-yl)-1,3-oxathiolane.(1S)-(+)-10-camphorsulfonate |

### EXAMPLES

The following examples are provided to illustrate various embodiments of the present invention and shall not be considered as limiting in scope.

### Example 1: Screening of chiral resolving agents

### Experimental conditions:

100 mg of cis-2-Hydroxymethyl-4-(cytosin-1'-yl)-1,3-oxathiolane is combined with 1 equivalent of the resolving agent, in 1 ml of solvent (95% ethanol- 5% water). The solid is isolated and weighed. A significant test requires that the weight of crystals does not exceed 50% of the overall diastereomer amount. If this condition is not fulfilled the amount or type of solvent is changed.

**TABLE 2**

| Chiral Resolving Agent | No crystal Observed | Crystal Observed |
|---|---|---|
| (-)-L-Malic acid | | x |
| (-)-L-Lactic acid | X (oil) | |
| (+)-L-Tartaric acid | | x |
| (-)-2,3-Dibenzoyl-L-Tartaric acid | | x |
| (1R)-(-)-10-Camphorsulfonic acid | | x |

### Example 2: Resolution experimentations

### General experimental conditions:

cis-2-Hydroxymethyl-4-(cytosin-1'-yl)-1,3-oxathiolane, and a chiral acid were dissolved with stirring in a solvent at a temperature of about room temperature to about 50 °C and then cooled at a temperature of between about room temperature to - 10°C for 2-4 hours. The solid product was collected by filtration. The composition was determined by Chiral HPLC, the aqueous buffer was prepared by diluting 0.5 ml triethylamine in 1L HPLC water, the pH adjusted to 6.88 with glacial acetic acid,. The mobile phase was prepared by combining the aqueous buffer and methanol in a ratio of 90:10 and the gas was removed. The following conditions were used:
Column: Astec Cyclobond I 2000 RSP, 5 micron, 250 x 4.5 mm.
Guard column: Astec Cyclobond I 2000 RSP, 20 x 4.0 mm Flow: 0.6 ml/min.
Sample preparation: prepare solution of 0.5 mg/ml in mobile phase.
Injection volume: 5 *µ*L.
Mode: isocratic.
UV-Vis detector art: 270 nm.
Column temperature: 0°C.
Run time: 40 min.

**TABLE 3**

| | Chiral resolving agent/achiral acid | Solvent | Experimental Conditions | Enantiome ric Ratio (yield %) |
|---|---|---|---|---|
| 1* | L-tartaric acid (0.5 eq)/HCl (0.5eq.) | Isopropyl alcohol and water 1:1 (v/v) | 100g scale. Magnetic stirring. | 45.2:52.1 (63%) |
| 2* | (R)-Camphorsulfonic Acid (0.5 eq)/HCl (0.5eq.) | Isopropyl alcohol and water 1:1 (v/v) | 5g scale. Heated @50°C to dissolve solids Magnetic stirring | 92.4:7.6 (36%) |
| 3* | (-)-2,3-Dibenzoyl-L-Tartaric acid (0.25eq)/HCl (0.5eq.) | Water | 5g scale. Magnetic stirring | 47.6:45.7 (41.7%) |
| 4 | (R)-Camphorsulfonic Acid (0.5 eq)/HCl (0.5eq.) | Isopropyl alcohol and water 1:1 (v/v) | 25g scale. Mechanical stirring | 90.9:9.1 (52.6%) |
| 5 | (R)-Camphorsulfonic Acid (0.5 eq)/HCl (0.5eq.) | Water Methanol 17% water in methanol (v/v) | 5g scale. Heated at reflux. Magnetic stirring | 78.7:21.3 (72%) |
| 6* | (R)-Camphorsulfonic Acid (1 eq)/no achiral acid | Isopropyl alcohol and water 1:1 (v/v) | 5g scale. Heated @50°C to dissolve solids Magnetic stirring | 53.6:46.4 (60.6%) |

| | | | | |
|---|---|---|---|---|
| * Reference examples | | | | |

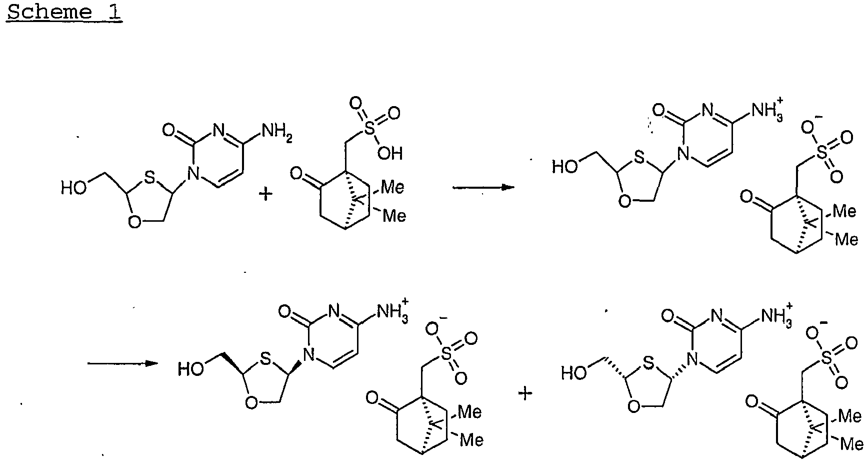

### Example 3: Diastereomeric salt optical resolution of cis-2-Hydroxymethyl-4-(cytosin-1'-yl)-1,3-oxathiolane. (reference example)

cis-2-Hydroxymethyl-4-(cytosin-1'-yl)-1,3-oxathiolane (1.03g, 4.3 mmol), and (1R)-(-)-10-Camphorsulfonic acid (1.03g, 4.3 mmol) were dissolved in 32 mL of a 1:1 isopropyl alcohol and water (v/v) at 50 °C. The solution was cooled at 0 °C. The solid was filtered to provide 0.55g of dry crystals. The diastereomeric composition was determined by HPLC to be 87:13 [(-)-cis-2-hydroxymethyl-4-(cytosin-1'-yl)-1,3-oxathiolane•(1R)-(-)-10-camphorsulfonic salt : (+)-cis-2-hydroxymethyl-4-(cytosin-1'-yl)-1,3-oxathiolane.(1R)-(-)-10-camphorsulfonic salt)].

The mother liquor was concentrated to dryness giving 1.38 g of dry solids with a diastereomeric composition of 35:65 [(-)-cis-2-hydroxymethyl-4-(cytosin-1'-yl)-1,3-oxathiolane•(1R)-(-)-10-camphorsulfonic salt : (+)-cis-2-hydroxymethyl-4-(cytosin-1'-yl)-1,3-oxathiolane•(1R)-(-)-10-camphorsulfonic salt)]. The composition was analyzed as shown in Example 2.

### Example 4: Recrystallization to increase the diastereomeric ratio of (-)-cis-2-hydroxymethyl-4-(cytosin-1'-yl)-1,3-oxathiolane•(1R)-(-)-10-camphorsulfonic salt with regard to (+)-cis-2-hydroxymethyl-4-(cytosin-1'-yl)-1,3-oxathiolane•(1R)-(-)-10-Camphorsulfonic salt. (reference example)

The crude cis-2-hydroxymethyl-4-(cytosin-1'-yl)-1,3-oxathiolane•(1R)-(-)-10-camphorsulfonate salt generated in entry 4 of table 3 in example 2 having an enantiomeric ratio of 90.9: 9.1 [(-)-cis-2-hydroxymethyl-4-(cytosin-1'-yl)-1,3-oxathiolane•(1R)-(-)-10-camphorsulfonic salt : (+)-cis-2-hydroxymethyl-4-(cytosin-1'-yl)-1,3-oxathiolane•(1R)-(-)-10-camphorsulfonic salt] was dissolved in isopropyl alcohol-water 1:1 (v/v) at 70°C. After cooling, the crystals were recovered with a yield of 76% and an enantiomeric ratio of 99.1: 0.9 [(-)-cis-2-hydroxymethyl-4-(cytosin-1'-yl)-1,3-oxathiolane•(1R)-(-)-10-camphorsulfonic salt : (+)-cis-2-hydroxymethyl-4-(cytosin-1'-yl)-1,3-oxathiolane•(1R)-(-)-10-camphorsulfonic salt].

### Example 5: Kilogram-scale Diastereomeric salt optical resolution

A mixture of isopropanol (2274 kg), distilled water (2905 kg), cis-2-Hydroxymethyl-4-(cytosin-1'-yl)-1,3-oxathiolane (193.7 kg) were introduced into the reactor (model R06 5700L GLMS). Dilute hydrochloric acid (prepared as 41.57 kg in 380 kg of water) was introduced followed by (1R)-(-)-10-camphorsulfonic acid (100 kg). The temperature of the resulting slurry was adjusted to 50 °C and agitated until all solid dissolved. The solution was then cooled to about -10°C (-13°C to -7°C) and agitated for 4-6 hours.

The resulting slurry is filtered, rinsing forward with 60 L of 1:1 isopropyl alcohol and water (v/v). The product is pulled dry with enantiomeric ratio of 91:9 [(-)-cis-2-hydroxymethyl-4-(cytosin-1'-yl)-1,3-oxathiolane•(1R)-(-)-10-camphorsulfonic salt : (+)-cis-2-hydroxymethyl-4-(cytosin-1'-yl)-1,3-oxathiolane•(1R)-(-)-10-camphorsulfonic salt)].

Isopropanol (228 kg) and water (291 kg) are added to the wet crude product then the temperature of the resulting solution was adjusted to 70°C and agitated until all solid dissolved the slurry is heated and agitated until all the solids dissolve. The solution is then cooled to about 22°C (19°C to 25°C) and then to 0°C (-3°C to 3°C).

The resulting slurry is filtered, rinsing forward with two portions of 70 L of 1:1 isopropyl alcohol and water (v/v). The product is spin-dried until the flow of filtrate essentially stops. 90.8 kg of product recovered (87% yield, corrected for loss on dryness of a sample) with an enantiomeric purity higher than 98%.

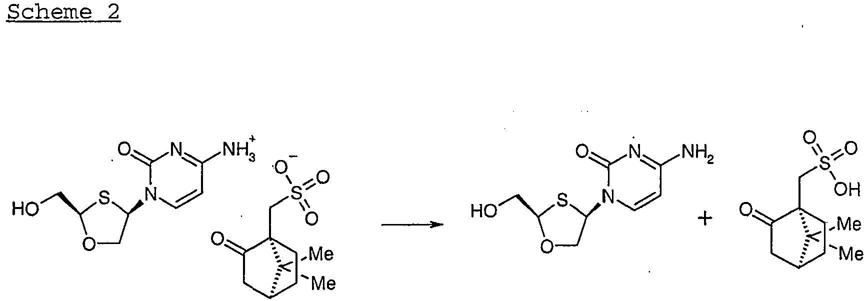

### Example 6: (1R)-(-)-10-camphorsulfonic acid removal from (-)-cis-2-hydroxymethyl-4-(cytosin-1'-yl)-1,3-oxathiolane•(1R)-(-)-10-camphorsulfonic salt (reference example)

(-)-cis-2-hydroxymethyl-4-(cytosin-1'-yl)-1,3-oxathiolane•(1R)-(-)-10-Camphorsulfonic salt (90.8 kg) is dissolved in methanol (601 kg) by heating the mixture at 40°C and agitating until a solution is achieved.

The warm solution is circulated through an ion exchange column containing Dowex® Marathon A-OH (133.6 kg) and methanol (200 kg) of while maintaining the temperature at 40°C until no residual camphorsulfonic acid is detected by NMR analysis and pH is greater than 7 (measured using water-wet pH paper).

The eluent is filtered, rinsing forward with methanol (200 kg).

The filtrate is partially concentrated under vacuum to about 140L.

The concentrate is cooled to about -10°C for one hour and agitated. The resulting slurry is filtered, rinsing forward with 2 portions of 18 kg of cold methanol (-10°C). The product is dried under vacuum while heating to 35-40°C.

The preceding examples can be repeated with similar success by substituting the generically or specifically described reactants and/or operating conditions of this invention for those used in the preceding examples.

## Claims

1. A process for producing crystalline optically active cis-2-hydroxymethyl-4-(cytosin-1'-yl)-1,3-oxathiolane, comprising:
a) reacting cis-2-hydroxymethyl-4-(cytosin-1'-yl)-1,3-oxathiolane with a chiral acid and an achiral acid to produce one diastereomeric salt and one enantiomeric salt;
b) recovering said diastereomeric salt;
c) converting said diastereomeric salt into optically active cis-2-hydroxymethyl-4-(cytosin-1'-yl)-1,3-oxathiolane,
wherein said chiral acid is (1R)-(-)-10-camphorsulfonic acid, and
wherein said diastereomeric salt is (-)-cis-2-hydroxymethyl-4-(cytosin-1'-yl)-1,3-oxathiolane.(1R)-(-)-10-camphorsulfonate.

2. The process according to claim 1, wherein said cis-2-hydroxymethyl-4-(cytosin-1'-yl)-1,3-oxathiolane is reacted with a half quantity molar amount of a chiral acid, said molar ratio being with regard to cis-2-hydroxymethyl-4-(cytosin-1'-yl)-1,3-oxathiolane.

3. The process according to claim 1 or 2, wherein in step a) a half-quantity molar amount of achiral acid is added to substantially produce (+)-cis-2-hydroxymethyl-4-(cytosin-1'-yl)-1,3-oxathiolane.achiral acid salt, said molar ratio being with regard to cis-2-hydroxymethyl-4-(cytosin-1'-yl)-1,3-oxathiolane.

4. The process according to any one of claims 1 to 3, wherein said achiral acid is hydrochloric acid.

5. The process according to any one of claims 1 to 4, wherein the solvent of the reaction is aqueous/organic, selected from methanol-water and isopropanol-water.

6. The process according to any one of claims 1 to 5, wherein the desired enantiomer is generated with an overall yield equal to or greater than 25%.

7. The process according to any one of claims 1 to 6, wherein the desired enantiomer is generated with an enantiomeric excess of 95% or higher.

8. The process according to any one of claims 1 to 7, wherein the desired enantiomer is generated with an overall yield equal to or greater than 25% and an enantiomeric excess of 99% or higher.

9. A compound selected from:
(-)-cis-2-hydroxymethyl-4-(cytosin-1'-yl)-1,3-oxathiolane•(1R)-(-)-10-camphorsulfonate;
(-)-cis-2-hydroxymethyl-4-(cytosin-1'-yl)-1,3-oxathiolane•(1S)-(+)-10-camphorsulfonate;
(+)-cis-2-hydroxymethyl-4-(cytosin-1'-yl)-1,3-oxathiolane.(1R)-(-) -10-camphorsulfonate; or
(+)-cis-2-hydroxymethyl-4-(cytosin-1'-yl)-1,3-oxathiolane•(1S)-(+)-10-camphorsulfonate.

## Patentansprüche

1. Verfahren zur Herstellung von kristallinem, optisch aktivem cis-2-Hydroxymethyl-4-(cytosin-1'-yl)-1,3-oxathiolan, umfassend:
a) Umsetzen von cis-2-Hydroxymethyl-4-(cytosin-1'-yl)-1,3-oxathiolan mit einer chiralen Säure und einer achiralen Säure, um ein diastereomeres Salz und ein enantiomeres Salz herzustellen;
b) Rückgewinnen des diastereomeren Salzes;
c) Umwandeln des diastereomeren Salzes in optisch aktives cis-2-Hydroxymethyl-4-(cytosin-1'-yl)-1,3-oxathiolan,
wobei die chirale Säure (1R)-(-)-10-Camphersulfonsäure ist und wobei das diastereomere Salz (-)-cis-2-Hydroxymethyl-4-(cytosin-1'-yl)-1,3-oxathiolan•(1R)-(-)-10-camphersulfonat ist.

2. Verfahren nach Anspruch 1, wobei das cis-2-Hydroxymethyl-4-(cytosin-1'-yl)-1,3-oxathiolan mit einer Hälfte der molaren Menge einer chiralen Säure umgesetzt wird, wobei das molare Verhältnis sich auf cis-2-Hydroxymethyl-4-(cytosin-1'-yl)-1,3-oxathiolan bezieht.

3. Verfahren nach Anspruch 1 oder 2, wobei in Schritt a) eine Hälfte der molaren Menge einer achiralen Säure zugegeben wird, um im Wesentlichen (+)-cis-2-Hydroxymethyl-4-(cytosin-1'-yl)-1,3-oxathiolan•achirales Säuresalz herzustellen, wobei das molare Verhältnis sich auf cis-2-Hydroxymethyl-4-(cytosin-1'-yl)-1,3-oxathiolan bezieht.

4. Verfahren nach einem der Ansprüche 1 bis 3, wobei die achirale Säure Salzsäure ist.

5. Verfahren nach einem der Ansprüche 1 bis 4, wobei das Lösungsmittel der Umsetzung wässrig/organisch ist, ausgewählt aus Methanol-Wasser und Isopropanol-Wasser.

6. Verfahren nach einem der Ansprüche 1 bis 5, wobei das gewünschte Enantiomer mit einer Gesamtausbeute gleich oder größer als 25% erzeugt wird.

7. Verfahren nach einem der Ansprüche 1 bis 6, wobei das gewünschte Enantiomer mit einem Enantiomerenüberschuss von 95% oder höher erzeugt wird.

8. Verfahren nach einem der Ansprüche 1 bis 7, wobei das gewünschte Enantiomer mit einer Gesamtausbeute gleich oder größer als 25% und einem Enantiomerenüberschuss von 99% oder höher erzeugt wird.

9. Verbindung, die ausgewählt ist aus:
(-)-cis-2-Hydroxymethyl-4-(cytosin-1'-yl)-1,3-oxathiolan•(1R)-(-)-10-camphersulfonat;
(-)-cis-2-Hydroxymethyl-4-(cytosin-1'-yl)-1,3-oxathiolan•(1S)-(+)-10-camphersulfonat;
(+)-cis-2-Hydroxymethyl-4-(cytosin-1'-yl)-1,3-oxathiolan•(1R)-(-)-10-camphersulfonat; oder
(+)-cis-2-Hydroxymethyl-4-(cytosin-1'-yl)-1,3-oxathiolan•(1S)-(+)-10-camphersulfonat.

## Revendications

1. Procédé de production de cis -2- hydroxyméthyl-4- (cytosin -1'- yl) -1,3-oxathiolane cristallin optiquement actif, comprenant :
a) la réaction du cis- 2-hydroxyméthyl- 4-(cytosin -1'- yl) -1,3- oxathiolane avec un acide chiral et un acide achiral pour produire un sel diastéréoisomère et un sel énantiomère ;
b) la récupération dudit sel diastéréoisomère ;
c) la conversion dudit sel diastéréoisomère en cis-2- hydroxyméthyl-4- (cytosin -1'-yl) -1,3- oxathiolane optiquement actif,
dans lequel ledit acide chiral est l'acide (1 R) - (-)-10-camphosulfonique , et dans lequel ledit sel diastéréoisomère est le (-)-cis- 2-hydroxyméthyl- 4-(cytosin -1'- yl) -1,3- oxathiolane • (1R)-(-)-10-camphorsulfonate

2. Procédé selon la revendication 1 , dans lequel ledit cis- 2-hydroxyméthyl- 4-(cytosin -1'- yl) -1,3- oxathiolane est mis à réagir avec une quantité molaire de la moitié de la quantité d'un acide chiral , ledit rapport molaire concernant le cis-2-hydroxy -4-(cytosine -1'- yl) -1,3 -oxathiolane.

3. Procédé selon la revendication 1 ou 2, dans lequel, dans l'étape a) une quantité molaire de la moitié de la quantité de l'acide achiral est ajoutée pour produire substantielllement le sel d'acide achiral •(+)-cis-2-hydroxyméthyl- 4-(cytosin-1'-yl) -1,3- oxathiolane, ledit rapport molaire concernant le cis- 2-hydroxyméthyl- 4-(cytosin -1'- yl)-1,3- oxathiolane.

4. Procédé selon l'une quelconque des revendications 1 à 3, dans lequel ledit acide achiral est l'acide chlorhydrique .

5. Procédé selon l'une quelconque des revendications 1 à 4, dans lequel le solvant de la réaction est aqueux / organique , choisi parmi le méthanol - eau et l'isopropanol - eau.

6. Procédé selon l'une quelconque des revendications 1 à 5, dans lequel l'énantiomère souhaité est produit avec un rendement global égal ou supérieur à 25%.

7. Procédé selon l'une quelconque des revendications 1 à 6, dans lequel l'énantiomère souhaité est produit avec un excès d'énantiomère de 95 % ou plus.

8. Procédé selon l'une quelconque des revendications 1 à 7, dans lequel l'énantiomère souhaité est produit avec un rendement global égal ou supérieur à 25% et un excès d'énantiomère de 99 % ou plus.

9. Un composé choisi parmi :
(-)-cis- 2-hydroxyméthyl- 4-(cytosin -1'- yl) -1,3- oxathiolane • (1R)-(-)-10-camphosulfonate ;
(-)-cis- 2-hydroxyméthyl- 4-(cytosin -1'- yl)-1,3- oxathiolane • (1S)-(+)-10-camphosulfonate ;
(+)-cis- 2-hydroxyméthyl- 4-(cytosin -1'- yl) -1,3- oxathiolane • (1R)-(-)-10-camphosulfonate , ou
(+)-cis- 2-hydroxyméthyl- 4-(cytosin -1'- yl) -1,3- oxathiolane • (1S)-(+)-10-camphosulfonate.
